# EUROPEAN PATENT APPLICATION

(11) **EP 4 278 963 A1**
(43) Date of publication of application: **22.11.2023**
(21) Application number: 22174309.9
(22) Date of filing: 19.05.2022
(51) Int. Cl.: A61B 5/0538, A61B 5/321, A61B 5/00

(54) **CONTROLLING ELECTRICAL SIGNALS BETWEEN A CATHETER AND A RECORDING SYSTEM**

(71) Applicant: Koninklijke Philips N.V., 5656 AG Eindhoven (NL)
(72) Inventor: VERSTRAELEN, Martinus Johannes Wilhelmina, Eindhoven (NL)
(74) Representative: Philips Intellectual Property & Standards

(57) **Abstract**

A mechanism for controlling signals between a first node, connected to a catheter electrode, and a second node, connected to a recording system. A signal processor processes an electrical signal provided to the first node by the catheter electrode. If a pacing signal is detected at the second node, a switching arrangement provides a bypass path between the first node and the second node, and the signal processor provides a steady state signal to the second node. If no pacing signal is detected at the second node, the bypass path is disabled and the signal processor provides a processed version of the electrical signal to the second node.

## Description

### FIELD OF THE INVENTION

The present invention relates to the field of catheters, and in particular to controlling electrical signals between a catheter and a recording system.

### BACKGROUND OF THE INVENTION

In the medical profession, the use of catheters carrying one or more electrodes ("catheter electrodes") is becoming more common. The catheter electrode(s) can be used to provide pacing signals to parts of a heart. It is becoming increasingly common to also use the electrode(s) to monitor electrical signals, e.g., electro-physiological signals such as electrocardiogram ("ECG") and in particular electrogram ("EKG") signals.

Typically, a same node is used to provide pacing signals for a catheter electrode as to provide sensed signal from the catheter electrode for recording. Thus, a recording system is usually able to route or generate the pacing signal as well as record the sensed signal. However, before being provided to said node for recordal by the recording system, the sensed signals undergo some interference (e.g., due to being used for other systems, such as catheter location detection systems).

This creates a conflict, as the interfered sensed signals (to be provided to the node) could interfere with the pacing signals provided to the node. There is a need to reduce or mitigate the impact of this interference.

US 8,406,875 B2 provides one approach for avoiding conflict between electrical signals sensed by an electrode of a catheter and pacing signals for the electrode of the catheter.

### SUMMARY OF THE INVENTION

The invention is defined by the claims.

According to examples in accordance with an aspect of the invention, there is provided a control system for controlling electrical signals between a catheter and a recording system.

The control system comprises: a first node for connecting to an electrode carried by the catheter; a second node for connecting to the recording system; a sensor configured to detect the presence or absence of one or more pacing signals, provided by the recording system for the catheter, at the second node; a switching arrangement configured to: complete a first electrical path between the first node and the second node responsive to the sensor detecting the presence of one or more pacing signals at the second node; and break the first electrical path between the first node and the second node responsive to the sensor detecting the absence of one or more pacing signals at the second node; and a second, different electrical path between the first node and the second node, the second electrical path comprising a signal processor for processing an electrical signal provided to the first node by the electrode carried by the catheter, the signal processor being configured to: provide the processed electrical signal to the second node responsive to the sensor detecting the absence of one or more pacing signals at the second node; and provide a steady state signal to the second node responsive to the sensor detecting the presence of one or more pacing signals at the second node.

The proposed embodiment provides a signal processor that performs the dual tasks of processing the electrical signal provided by a catheter electrode and preventing the processed electrical signal from interfering or disrupting a pacing operation. This facilitates performance of a dual functionality of the signal processor.

The steady state signal provided by the signal processor also acts as a driving signal, avoiding a floating output connection to the second node. This reduces or avoids potential erratic behavior at the second node. In particular, the electrical reference at the second node can be preserved during pacing.

In some examples, the signal processor is configured to perform a noise reduction technique on the electrical signal in producing the processed electrical signal. This provides a useful processing of the electrical signal that would contribute to improved utility and/or reduced error in the electrical signal.

The signal processor may be configured to perform power-line interference noise reduction on the electrical signal in producing the processed electrical signal. Power-line interference (PLI) is a significant problem with electrical signals sensed by catheter electrodes, and especially cathetres for sensing cardiac signals representing electrical activity of the heart for example by measuring Electrogram (EKG) signals. It has been recognized that PLI can significantly affect the interpretability of, for instance, cardiac signals. Power line interference typically has a fundamental frequency of around 50Hz/60Hz (jurisdiction dependent), which falls within the range of standard or conventional signals monitored by a catheter electrode (e.g., ECG and/or EKG signals between 0.05Hz and 150Hz or 0 and 100Hz). PLI reduction is therefore particularly useful for use in processing electrical signals obtained by a catheter electrode.

The steady state signal may be a direct current signal. This approach provides a constant value at the second node for reducing unexpected or undesirable behavior of element connected to the second node. A direct current signal, acting as the steady state signal, acts as an electrical reference for the second node, reducing unexpected behavior.

Preferably, the steady state signal provides no or negligible power. This will further reduce the impact of the output of the signal processor on the pacing signals provided to the catheter electrode via the first node.

In some examples, the second electrical path further comprises a resistive element connected between the signal processor and the second node. This approach further reduces potential interference or distortion of the pacing signal(s) by the output of the signal processor, as the resistive element will reduce current flow from the second node to the signal processor (as there is a direct connection from the second node to the first node).

The resistive element may have a greater resistance than a parasitic resistance for a hypothetical direct wire connection between the signal processor and the second node. Thus, the resistive element may not be a simple wire connection between the signal processor and the second node.

In some examples, the second electrical path further comprises an intervening system connected between the first node and the signal processor.

The intervening system may comprise a position tracking system for determining a position of the catheter responsive to the electrical signal provided to the first node. Other modules for use in a suitable intervening system for analyzing or using the electrical signals from the catheter electrode (or providing signals to the catheter electrode) could be used, such as an ablation system and/or a contact sensing system.

There is also proposed an apparatus comprising a herein described and/or claimed control system and a recording system configured to controllably provide pacing signals to the second node of the control system.

The apparatus may comprise the catheter comprising the electrode configured to generate an electrical signal responsive to changes in electrical potential at the electrode.

The apparatus may be or comprise a cardiac mapping system. Such system is capable of providing information to a user on cardiac activity. The apparatus can be or comprise one known as Carto^{™}, EnSite^{™}, Rythmia^{™}, Kodex^{™} or other.

There is also proposed a method for controlling electrical signals between a catheter and a recording system using a control system comprising a first node for connecting to an electrode carried by the catheter; and a second node for connecting to the recording system.

The method comprises: using a sensor to detect the presence or absence of one or more pacing signals, provided by the recording system for the catheter, at the second node; using a switching arrangement to complete a first electrical path between the first node and the second node responsive to detecting the presence of one or more pacing signals at the second node; using the switching arrangement to break the first electrical path between the first node and the second node responsive detecting the absence of one or more pacing signals at the second node; using a signal processor for processing an electrical signal provided to the first node by the electrode carried by the catheter; provide the processed electrical signal to the second node responsive to the sensor detecting the absence of one or more pacing signals at the second node; and provide a steady state signal to the second node, being a signal that avoids interfering with the one or more pacing signals, responsive to the sensor detecting the presence of one or more pacing signals at the second node.

Processing the electrical signal may comprise performing a noise reduction technique on the electrical signal. The noise reduction technique may be a power-line interference noise reduction technique.

The steady state signal is a direct current signal.

The steady state signal preferably provides no or negligible power.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a control system;
Figure 2 illustrates a control system according to an embodiment; and
Figure 3 is a flowchart illustrating a method according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the claims. These and other features, aspects, and advantages of the apparatus, systems and methods of the present disclosure will become better understood from the following description, appended claims, and accompanying drawings. The Figures are merely schematic and are not drawn to scale. The same reference numerals are used throughout the Figures to indicate the same or similar parts.

The disclosure provides a mechanism for controlling signals between a first node, connected to a catheter electrode, and a second node, connected to a recording system. A signal processor processes an electrical signal provided to the first node by the catheter electrode. If a pacing signal is detected at the second node, a switching arrangement provides a bypass path between the first node and the second node, and the signal processor provides a steady state signal to the second node. If no pacing signal is detected at the second node, the bypass path is disabled, or made into an open circuit, and the signal processor provides a processed version of the electrical signal to the second node.

Embodiments are based on the realization that a signal processor is operable in two modes, one mode in which an electrical signal, at a first node, can be processed and provided to a recording system at a second node and another mode in which a steady state signal can be provided to avoid interfering with any pacing signals provided at the second node. Embodiments are also based on the realization that a steady state signal will provide negligible disruption or distortion to any pacing signals.

Approaches proposed by the present disclosure can be adopted in any suitable environment in which communication between a catheter and a recording system is required and includes the passing of pacing signals to an electrode of a catheter. Embodiments are particular advantageous in the assessment and treatment of heart conditions.

In the context of the present disclosure, an "open" switch is one that has been controlled to become conductive, i.e., designed to conduct electricity. A "closed" switch is one that has been controlled to become non-conductive, i.e., designed to not conduct electricity.

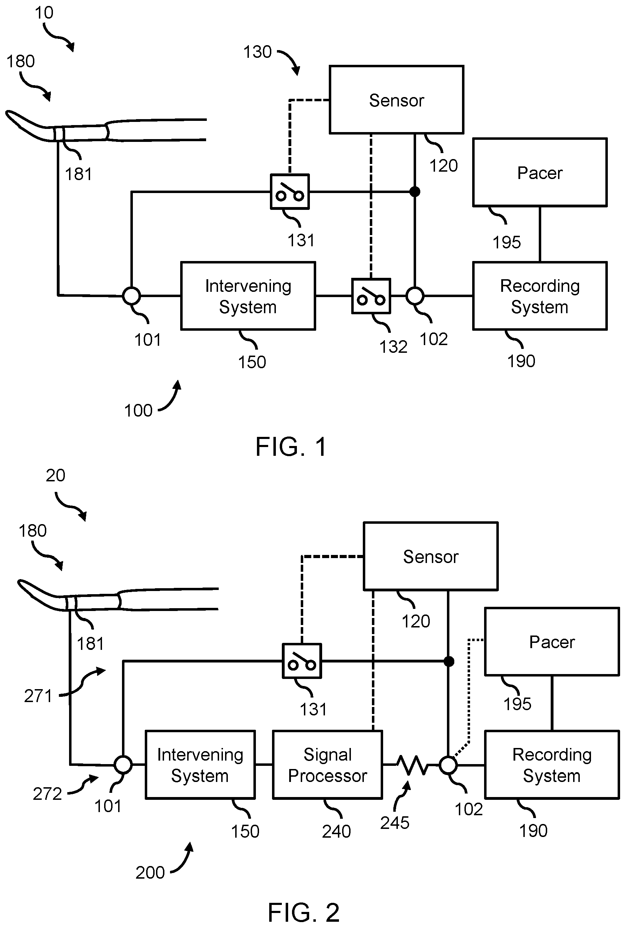

Figure 1 illustrates an existing control system 100 for improved contextual understanding. The control system is illustrated in the context of an apparatus 10, which comprises the control system 100, a catheter 180 and a recording system 190.

The control system 100 is configured to control the signals communicated between a first node 101 and a second node 102. The first node 101 is for connection to an electrode 181 on the catheter 180, also known as a catheter electrode 181. The second node 102 is for connection to the recording system 190.

The catheter electrode 181 is configured to pass an electrical signal to the first node 101. The electrical signal may, for instance, comprise an electrophysiological signal, such as a cardiac signal. The electrical signal may represent an electrical potential sensed by the catheter electrode 181, e.g., in the vicinity of a subject's heart. Of course, the precise nature of the electrical signal will depend upon at least the location of the catheter 180 with respect to the subject.

Usually, the electrical signal is routed by the control system 100 from the first node 101 to the second node 102.

There may be an intervening system 150 between the first node 101 and the second node 102 that makes use of the electrical signal. For instance, an intervening system may comprise a position sensing system, configured to determine the position of the catheter electrode 181 and/or the catheter 180 using the electrical signal.

The recording system 190 receives the electrical signal at the first node, and stores the value(s) of the electrical signal, e.g., performs sampling of the routed electrical signal to store information about the electrical signal.

The apparatus 10 is further configured so that a pacing signal, e.g., for performing pacing of the heart, can be provided to the same catheter electrode 181. A pacing signal may be generated by a pacer 195 and routed to the second node 102 via the recording system 190. Any suitable pacer for generating pacing signals may be used, and well known-examples will be apparent to the skilled person. One example of a pacing system for use as a pacer is described by the US patent application having publication number US 3,815,611 A.

There is a need to route the pacing signal from the second node 102 to the first node 101 (and therefore to the connected catheter electrode 181). However, the intervening system 150 may introduce unwanted noise or distortion to the pacing signal if the pacing signal were to pass therethrough.

The control system 100 is configured to controllably bypass the intervening system 150 to avoid distortion of the pacing signal by the intervening system.

A sensor 120 of the control system 100 is configured to identify time intervals in which pacing signals are transmitted by the recording device 190, i.e., detect the presence or absence of a pacing signal at the second node 102. The sensor may comprise or consist of hardware, optionally in combination with drivers in the form of e.g. software. Preferably, the hardware comprises circuitry for signal processing and wiring for connectivity. Optionally signal processing may at least partially be implemented in software form.

Control circuitry 130 of the control system 100 comprises a first switch 131 and a second switch 132. The first switch 131 connects between the first node 101 and the second node 102. The second switch 132 connects between the intervening system 150 and the second node 102.

If the sensor 120 detects the presence of a pacing signal at the second node, the first switch 131 is closed and the second switch 132 is opened. The pacing signal thereby passes directly from the second node 102 to the first node 101, bypassing the intervening system 150.

If the sensor 120 detects the absence of a pacing signal at the second node, the first switch 131 is opened and the second switch 132 is closed. The electrical signal provided by the electrode 181 of the catheter 180 is therefore passed to the second node via the intervening system 150. The direct connection between the first node 101 and second node 102 (via the first switch 131) is not made.

Figure 2 illustrates a control system 200 according to an embodiment. As before, the control system 200 is illustrated in the context of an apparatus 20, which comprises the control system 200, a catheter 180 and a recording system 190.

As before, a catheter electrode 181 of the catheter 180 provides an electrical signal to a first node 101 of the control system 200. Similarly, one or more pacing signals may be provided to the second node 102 of the control system, e.g., via a pacer 195 that provides the pacing signals via the recording system 190. Alternatively, the pacer 195 may be configured to directly provide the pacing signal(s) to the second node 102, i.e., so that the pacing signal is not routed via the recording system 190.

The control system 200 is configured to route an electrical signal received at the first node 101 to the second node 102 (and thereby to the recording system 190) and route any pacing signals provided at the second node 102 to the first node 101 (and thereby to the catheter electrode 181).

The control system 200 differs from the control system previously described in that the second switch 132 is replaced by a signal processor 240. The signal processor 240 is configured to process the electrical signal provided to the first node 101 by the electrode 181 carried by the catheter 180.

The sensor 120 is still configured to detect the presence or absence of a pacing signal at the second node 102. Approaches for detecting the presence or absence of a pacing signal are well known in the art. For instance, the sensor 120 may monitor the second node 102 to detect a voltage and/or current exceeding a predetermined threshold and/or meeting a predetermined pattern (representative of a pacing).

The precise sensing performed by the sensor 120 may depend upon the nature of the pacing performed by the pacer 195. For instance, different pacers 195 may (when pacing) provide different patterns in a signal. The sensor 120 may be configured to detect the pattern, indicative of a pacing, in a signal at the second node.

As before, a first switch 131 is configured to close and connect the first node 131 to the second node 132 responsive to the sensor 120 detecting the presence of a pacing signal at the second node. In this way, the pacing signal(s) are able to effectively bypass the signal processor.

Similarly, the first switch 131 is configured to open responsive to the sensor 120 detecting the absence of a pacing signal at the second node. Opening the first switch 131 prevents or avoids the output of the signal processor from being fed back to the catheter and/or the input of the signal processor.

The first switch 131 may be formed from any suitable switch for use in conducting electricity, such as a MOSFET or similar.

Thus, there is provided a switching arrangement 131 (e.g., comprising the first switch) that completes a first electrical path 271 between the first node 101 and the second node 102 responsive to the sensor 120 detecting the presence of one or more pacing signals at the second node; and breaks the first electrical path between the first node and the second node responsive to the sensor detecting the absence of one or more pacing signals at the second node. The switching arrangement can comprise drivers and/or biasing circuitry required for controlling a gate or base voltage of a switch such as for example a transistor, MOSFET, BJT or JFET.

The signal processor 240 forms part of a second electrical path 272 from the first node 101 to the second node 102. The second electrical path 272 differs from the first electrical path 271.

The signal processor 240 is configured to provide the processed electrical signal to the second node responsive to the sensor detecting the absence of one or more pacing signals at the second node.

The signal processor 240 is also configured to provide a steady state signal (e.g., having a constant signal value) to the second node 102 responsive to the sensor detecting the presence of one or more pacing signals at the second node. Thus, there is no physical disconnection of the signal processor from the second node. Such a steady state signal will not interfere with the propagation of the pace signal.

The steady state signal may, for instance, be a direct current (DC) signal. It has been identified that provision of such a steady state signal will have no or negligible impact on the pacing signal(s) provided to the second node. In particular, the steady state signal will act like an electrical reference.

The steady state signal may also have a steady or near-constant output impedance, e.g., to provide a steady impedance state to the second node 102. This ensures a steady impedance is provided to the catheter electrode 181 (via the closed first switch 131 and the first node 101) for reduced error in performing pacing or the like.

Thus, the steady state signal may provide a direct current signal (e.g., a constant voltage and/or current) with a steady or near-constant output impedance. In this way, the signal processor can be configured to act in a similar manner to a DC voltage supply. This approach means that an electrical reference for the second node is preserved during pacing activity, reducing potential error or unexpected behavior, e.g., of the recording system 190 that samples or measures a voltage at the second node and/or by the catheter electrode.

Thus, rather than providing a floating output to the second node (i.e., by opening the second switch 132 of the control circuitry 130 of control system 100), a steady state signal is provided to the second node when a pacing signal is detected. The presence of a floating output can cause erratic or unpredictable behavior, which is avoided or attenuated using the proposed approach.

The proposed system provides a mechanism by which a signal processor can perform a dual task of processing the electrical signal provided by the catheter electrode 181 and preventing the processed electrical signal from disrupting or distorting the pacing signal(s). This reduces an amount of circuitry and/or control signals required to perform a dual function of processing and avoidance of pacing signal distortion.

The control system thereby effectively operates in two modes. In a first mode, an electrical signal, at the first node is processed by the signal processor and provided to the second node. In a second mode, the second node is connected to the first node via a switching arrangement, e.g., to bypass as least the signal processor, and the signal processor provides a steady state signal to the second node avoid interfering with or distorting any pacing signals provided at the second node.

The control system operates in the first mode (only) if/when no pacing signals are present or detectable at the second node. The control system operates in the second mode (only) if/when any pacing signals are present or detectable at the second node

The signal processor may be configured to perform a noise reduction technique, e.g., noise filtering or noise attenuation technique, on the electrical signal provided by the catheter electrode 181.

The processing performed by the signal processor 240 may be performed in the analogue and/or digital domain. The signal processor may, where appropriate, therefore comprise one or more analogue-to-digital converters and/or digital-to-analogue converters.

A signal processor that operates in the digital domain provides a mechanism that is ready capable of responding to an indication (from the sensor) that one or more pacing signals have been detected and outputting a steady state signal. However, suitable approaches for performing this operation using purely analogue components would be apparent to the skilled person.

In particularly preferable examples, the signal processor is configured to perform power-line interference noise reduction on the electrical signal in producing the processed electrical signal. PLI can significantly affect the interpretability of, for instance, cardiac signals.

Suitable approaches are known in the art, such as that disclosed by Manosueb, Anchalee, Jeerasuda Koseeyaporn, and Paramote Wardkein. "PLI cancellation in ECG signal based on adaptive filter by using Wiener-Hopf equation for providing initial condition." Computational and mathematical methods in medicine 2014 (2014) or Koseeyaporn, Poolsak, Jeerasuda Koseeyaporn, and Paramote Wardkein. "An enhanced adaptive algorithm for PLI cancellation in ECG signals." 2009 7th International Conference on Information, Communications and Signal Processing (ICICS). IEEE, 2009.

The first electrical path 271, which is completed when a pacing signal is detected at the second node, will ensure that the electrical signal generated by the catheter electrode 181 is still provided to the recording system, albeit of a reduced quality and/or partially distorted. This is because any processing of the signal by the signal processor 240 will not be present in the version of the electrical signal received by the recording system (as it is instead providing the steady state signal).

It is noted that the electrical signal may carry useful information at frequencies other than the pacing signal, such that the effects of the pacing signal on the electrical signal can be filtered out (especially as the characteristics of the pacing signal can be known, e.g., if the recording system also records the pacing signal) in future processing techniques.

Optionally, the second electrical path 272 further comprises a resistive element 245 connected between the signal processor and the second node. The resistive element 245 has a non-infinite resistance, e.g., has a resistance of less than 100MΩ, e.g. less than 1MΩ. The resistive element 245 acts to further reduce an impact of the processed electrical signal and reduce any potential leakage of the pacing signal to the signal processor.

Preferably, the resistive element has a greater resistance than a parasitic resistance for a hypothetical direct wire connection between the signal processor and the second node. This approach will achieve a reduction in reduce the impact of the processed electrical signal and reduce any potential leakage of the pacing signal to the signal processor.

For instance, the resistive element 245 may have a resistance greater than 10Ω, for instance, greater than 50Ω.

As before, the apparatus 20 may comprise an intervening system 150. The intervening system may be connected between the first node 101 and the signal processor 240.

The intervening system may, for instance, comprise a position tracking system for determining a position of the catheter responsive to the electrical signal provided to the first node. Suitable approaches for determining or monitoring a position of a catheter electrode and/or catheter using an electrical signal monitored by the catheter electrode are disclosed by the European Patent Applications having publication numbers EP 0775466 A2, EP 3568068 A1 and EP 3607879 A1. Any of these approaches, or others established in the art, could be employed.

Other suitable examples of components or modules for use in an intervening system will be apparent to the skilled person. For instance, the intervening system may comprise an ablation system, configured to control an ablation signal provided to the electrode connected to the first node 101. As another example, the intervening system may comprise a contact sensor configured to monitor the electrical signal provided to the first node to predict whether the catheter electrode 180 makes or has made contact with an anatomical element of a subject.

The skilled person will appreciate that the illustrated control system is described in the context of a catheter supporting a single catheter electrode. However, approaches may be readily adapted for catheters having multiple electrodes, e.g., no fewer than six electrodes or no fewer than twenty electrodes. Each electrode may be connected to a respective (and different) first node of such a control system, and the control system may be configured to control communication between such first nodes and respective second nodes (for each electrode) for connection to the recording system.

Thus, there may be a plurality of sets of first electrical paths and second electrical paths, each set being associated with a different electrode of the catheter.

Put another way, the illustrated example provides a single channel connection between the catheter and the recording system. However, embodiments may comprise a plurality of channels between the catheter and the recording system, each channel representing a different electrode of the catheter.

The catheter 180 is an intravenous catheter, sized and configured for entering one or more anatomical cavities (e.g., lumens) of a subject. The catheter carries one or more electrodes, including at least the electrode connected to the first node 101. Example catheters are Electrophysiology catheters (also known as EP catheters) and RF-, PFA and other ablation catheters. Electrical signals present at the electrode represent electrical activity near the electrode, which may be caused by electrical activity of the subject (e.g., cardiac signals) and/or induced in the subject by another source, e.g., the electrode itself, another electrode of the catheter and/or an electrode positioned externally to the subject. Such electrical signals carry useful information for assessing the condition of the subject and/or determining characteristics (e.g., position, whether or not contact is made etc.) of the catheter.

The recording system 190 may comprise a memory or storage unit configured to store electrical signals receive at the second node. The recording system 190 may also record pacing signals provided by the pacer 195. Further components may be able to access the recording system, e.g., to extract data stored by the recording system 190.

Further possible components of an apparatus are not illustrated for the sake of clarity. For instance, the apparatus may comprise a display system configured to provide a visual representation of signals present at the second node. In some examples, the apparatus may comprise further processing circuitry, e.g., configured to receive signals recorded by the recording system, for analysis and/or conversion to a visual format for display at a user interface.

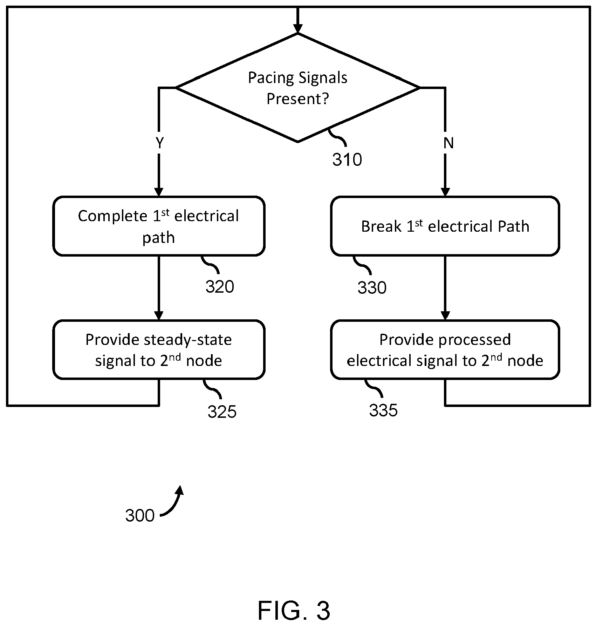

Figure 3 illustrates a method 300 for controlling electrical signals between a catheter and a recording system using a control system comprising a first node for connecting to an electrode carried by the catheter; and a second node for connecting to the recording system.

The method comprises a step 310 of detecting the presence or absence of one or more pacing signals, provided by the recording system for the catheter, at the second node.

Step 310 may be performed, for instance, by obtaining (at the sensor) a sensing signal representing or responsive to a signal at the second node. The obtained sensing signal may then be processed to predict whether or not the pacing signal(s) are present at the second node.

Responsive to the step 310 detecting the presence of one or more pacing signals at the second node, the method 300 performs a step 320 of using a switching arrangement to complete a first electrical path between the first node and the second node.

Responsive to the step 320 detecting the presence of one or more pacing signals at the second node, the method 300 also performs a step 325 of using a signal processor (for processing an electrical signal provided to the first node by the electrode carried by the catheter) to provide a steady state signal to the second node. The steady state signal is a signal that avoids interfering with the one or more pacing signals.

Responsive to the step 310 detecting the absence of one or more pacing signals at the second node, the method performs a step 330 of using the switching arrangement to break the first electrical path between the first node and the second node.

Responsive to the step 310 detecting the presence of one or more pacing signals at the second node, the method 300 also performs a step 335 of using the signal processor (for processing an electrical signal provided to the first node by the electrode carried by the catheter) to provide the processed electrical signal to the second node responsive to the sensor.

The electrical signal may, for instance, only be processed by the signal processor responsive to detecting the absence of one or more pacing signals at the second node. However, this is not essential.

The method 300 may be iteratively repeated, e.g., until stopped via an override signal which may represent a user indication to stop.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A control system for controlling electrical signals between a catheter and a recording system, the control system comprising:
- a first node for connecting to an electrode carried by the catheter;
- a second node for connecting to the recording system;
- a sensor configured to detect the presence or absence of one or more pacing signals, provided by the recording system for the catheter, at the second node;
a switching arrangement configured to:
complete a first electrical path between the first node and the second node responsive to the sensor detecting the presence of one or more pacing signals at the second node; and
break the first electrical path between the first node and the second node responsive to the sensor detecting the absence of one or more pacing signals at the second node; and
a second, different electrical path between the first node and the second node, the second electrical path comprising a signal processor for processing an electrical signal provided to the first node by the electrode carried by the catheter, the signal processor being configured to:
provide the processed electrical signal to the second node responsive to the sensor detecting the absence of one or more pacing signals at the second node; and
provide a steady state signal to the second node responsive to the sensor detecting the presence of one or more pacing signals at the second node.

2. The control system of claim 1, wherein the signal processor is configured to perform a noise reduction technique on the electrical signal in producing the processed electrical signal.

3. The control system of claim 2, wherein the signal processor is configured to perform power-line interference noise reduction on the electrical signal in producing the processed electrical signal.

4. The control system of any of claims 1 to 3, wherein the steady state signal is a direct current signal.

5. The control system of any of claims 1 to 4, wherein the steady state signal provides no or negligible power.

6. The control system of any of claims 1 to 5, wherein the second electrical path further comprises a resistive element connected between the signal processor and the second node.

7. The control system of claim 6, wherein the resistive element has a greater resistance than a parasitic resistance for a hypothetical direct wire connection between the signal processor and the second node.

8. The control system of any of claims 1 to 7, wherein the second electrical path further comprises an intervening system connected between the first node and the signal processor.

9. The control system of claim 8, wherein the intervening system comprises a position tracking system for determining a position of the catheter responsive to the electrical signal provided to the first node.

10. An apparatus comprising:
the control system of any of claims 1 to 9;
a recording system configured to controllably provide pacing signals to the second node of the control system.

11. The apparatus of claim 10, further comprising the catheter comprising the electrode configured to generate an electrical signal responsive to changes in electrical potential at the electrode.

12. A method for controlling electrical signals between a catheter and a recording system using a control system comprising a first node for connecting to an electrode carried by the catheter; and a second node for connecting to the recording system, the method comprising:
using a sensor to detect the presence or absence of one or more pacing signals, provided by the recording system for the catheter, at the second node;
using a switching arrangement to complete a first electrical path between the first node and the second node responsive to detecting the presence of one or more pacing signals at the second node;
using the switching arrangement to break the first electrical path between the first node and the second node responsive detecting the absence of one or more pacing signals at the second node;
using a signal processor for processing an electrical signal provided to the first node by the electrode carried by the catheter:
provide the processed electrical signal to the second node responsive to the sensor detecting the absence of one or more pacing signals at the second node; and
provide a steady state signal to the second node, being a signal that avoids interfering with the one or more pacing signals, responsive to the sensor detecting the presence of one or more pacing signals at the second node.

13. The method of claim 12, wherein processing the electrical signal comprises performing a noise reduction technique on the electrical signal, such as a power-line interference noise reduction technique.

14. The method of any of claims 12 to 13, wherein the steady state signal is a direct current signal.

15. The method of any of claims 12 to 14, wherein the steady state signal provides no or negligible power.
